(19) Europäisches Patentamt
European Patent Office
Office européen des brevets

(11) Veröffentlichungsnummer: **0 013 252**
**B1**

(12) **EUROPÄISCHE PATENTSCHRIFT**

(45) Veröffentlichungstag der Patentschrift: **23.09.87**

(51) Int. Cl.⁴: **C 07 D 233/52,** C 07 D 405/12, A 61 K 31/415

(21) Anmeldenummer: **79710010.4**

(22) Anmeldetag: **09.11.79**

# (54) Mittel zur Senkung der Herzfrequenz.

(30) Priorität: **21.12.78 DE 2855306**

(43) Veröffentlichungstag der Anmeldung: **09.07.80 Patentblatt 80/14**

(45) Bekanntmachung des Hinweises auf die Patenterteilung: **23.09.87 Patentblatt 87/39**

(84) Benannte Vertragsstaaten: **AT BE CH DE FR GB LU NL SE**

(56) Entgegenhaltungen:
**DE-A-2 457 979**

**Arzneimittelforschung, Bd. 16, Nr. 8 (1966), Seiten 1038-1050**
**Arzneimittelforschung, Bd. 25, Nr. 5 (1975) Seiten 786-793**
**Arch. int. Pharmacodyn. Bd. 228 (1977) Seiten 237-250**
**Arch. int. Pharmacodyn Bd. 228 (1977) Seiten 251-267**
**Hypertension and Brain Mechanisms. Progress in Brain Research. W. de Jong A.P. Provost and A.P. Shapiro, Bd. 47 (1977) Seiten 391-396**

**Die Akte enthält technische Angaben, die nach dem Eingang der Anmeldung eingereicht wurden und die nicht in dieser Patentschrift enthalten sind.**

(73) Patentinhaber: **C.H. BOEHRINGER SOHN, Postfach 200, D-6507 Ingelheim am Rhein (DE)**

(72) Erfinder: **Stähle, Helmut, Dr., Rotweinstrasse 23, D-6507 Ingelheim/Rhein (DE)**
Erfinder: **Köppe, Herbert, Dr., Neuweg 72, D-6507 Ingelheim/Rhein (DE)**
Erfinder: **Kummer, Werner, Dr., Georg- Scheuing-Strasse 15, D-6507 Ingelheim/Rhein (DE)**
Erfinder: **Kobinger, Walter, Prof. Dr., Belghofergasse 27, A-1120 Wien (AT)**
Erfinder: ***Lillie, Christian,* Dr. Mag., Hansi- Niese-Weg 12, A-1130 Wien (AT)**
Erfinder: **Pichler, Ludwig, Dr., Gusshausstrasse 24, A-1040 Wien (AT)**
Erfinder: **Hoefke, Wolfgang, Dr., Schillerstrasse 13, D-6501 Budenheim (DE)**
Erfinder: **Gaida, Wolfram, Dr., Bahnhofstrasse 74, D-6507 Ingelheim/Rhein (DE)**

EP 0 013 252 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

In DE-A-24 57 979 werden 2-N-Aryl-hydroxyamino-imidazoline-(2) der allgemeinen Formel

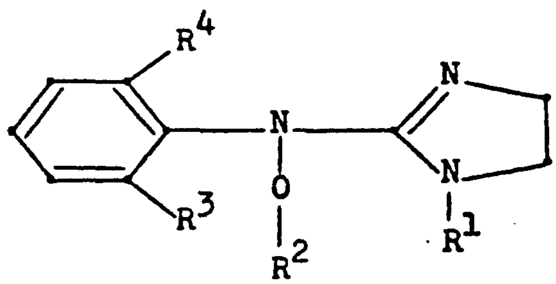

I

worin
$R^1$ Wasserstoff oder eine niedere Alkylgruppe,
$R^2$ Wasserstoff, eine niedere Alkyl-, niedere Hydroxyalkyl-, Chlor-hydroxypropyl-, 2,3-Epoxypropyl-, Alkoxyalkyl-, Aminoalkyl-, N-substituierte Aminoalkyl-, Benzyl- oder Phenäthylgruppe,
$R^3$ Wasserstoff oder Halogen und
$R^4$ Halogen oder Methyl
bedeutet und deren Säureadditionssalze sowie ein Verfahren zus Herstellung beschrieben. Die Vorveröffentlichung lehrt, daß diese Verbindungen sich in erster Linie durch eine antihypertensive Wirkung auszeichnen und zur Herstellung von Lösungen für Injektionszwecke und insbesondere von peroral zu verabreichenden pharmazeutischen Präparaten zur Behandlung der Hypertonie verwendet werden können.

Es wurde nun überraschenderweise gefunden, daß die Verbindungen der allgemeinen Formel I und deren Säureadditionssalze eine deutliche Senkung der Herzfrequenz bewirken. Sie eignen sich damit besonders zur Prophylaxe und Therapie ischämischer Herzerkrankungen und von Sinustachykardien verschiedener Genese.

Gegenstand der Erfindung ist die Verwendung von 2-N-Phenyl-hydroxy-amino-imidazolinen-(2) der allgemeinen Formel

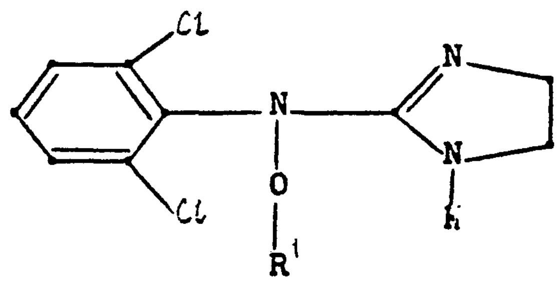

I

in der
$R^1$ eine Methyl-, Propyl- oder Isobutylgruppe, Hydroxyäthyl-, Hydroxypropyl-, Chlor-hydroxypropyl-, Methoxyalkyl-, Benzyl- oder Phenäthylgruppe,
bedeutet sowie von deren physologisch verträglichen Säureadditionssalzen zur Herstellung eines Arzneimittels zur Behandlung ischämischer Herzerkrankungen und vom Sinustachykardien verschiedener Genese.

2-[N-(2,6-Dichlorphenyl)-methoxyamino]-imidazolin-(2) wurde an narkotisierten Tieren untersucht. An der Katze war nach intravenöser Gaben von 0,3 und 1,0 mg/kg eine deutliche dosisabhängige Bradykardie zu verzeichnen. An der intakten Ratte wurde nach 0,6 mg/kg der Substanz zunächst eine Steigerung des Blutdrucks um 5 mm Hg, denn eine Senkung um 15 mm Hg beobachtet. Daneben erfolgte eine Senkung der Herzfrequenz um 58 Schläge je Minute. Bei einer Dosis 2,5 mg/kg trat bei einer Blutdruckveränderung von +22 auf -39 Hg eine Herzfrequenzsenkung um 145 Schläge je Minute ein.

Die Substanz war nach Ausschaltung des ZNS an der Ratte voll bradykard wirksam. An der Spinalratte bewirkten 0,6 mg/kg eine Herzfrequenzsenkung um 70 Schläge je Minute und 2,5 mg/kg eine Senkung um 140 Schläge pro Minute. Dies spricht für einen direkten Angriffspunkt am Herzen. Die Ergebnisse grenzen die Verbindung deutlich von anderen bradykard wirksamen Substanzen und Substanzgruppen, wie Clonidin, Calciumantagonisten vom Verapamiltyp, cholinergen Substanzen, β-Arenorezeptorenblocker, ab.

In der gleichen Weise wirken die übrigen Verbindung der allgemeinen Formel I. In der folgenden Tabelle sind für eine Auswahl der Verbindungen die Dosen vermerkt, die an der Spinalratte eine Senkung der

Herzfrequenz um 150 Schläge je Minute (D 150-Wert) bewirken.

| Verbindung der Formel I (Basen) | | | | D 150 |
|---|---|---|---|---|
| $R^1$ | $R^2$ | $R^3$ | $R^4$ | mg/kg |
| H | $C_6H_5CH_2$- | Cl | Cl | 1,05 |
| H | $C_3H_7$- | Cl | Cl | 1,3 |
| H | $(CH_3)_2CHCH_2$- | Cl | Cl | 2,3 |
| H | $HOCH_2CH_2$- | Cl | Cl | 1,3 |
| H | $CH_3OCH_2CH_2$- | Cl | Cl | 1,5 |
| H | $C_6H_5CH_2CH_2$- | Cl | Cl | 2,6 |
| H | $ClCH_2CHOHCH_2$ | Cl | Cl | 3,4 |
| H | $HO(CH_2)_3$- | Cl | Cl | 4,0 |
| H | $CH_3CHOHCH_2$ | Cl | Cl | 5,7 |

Die Verbindungen der allgemeinen Formel I können in die üblichen galenischen Anwendungsformen wie Tabletten, Dragées, Lösungen, Emulsionen, Pulver, Kapseln oder Depotformen gebracht werden, wobei zu deren Herstellung die üblichen pharmazeutischen Hilfsstoffe sowie die üblichen Fertigungsmethoden herangezogen werden können. Entsprechende Tabletten können beispielsweise durch Mischen der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung eines Depoteffekts, wie Carboxypolymethylen, Carboxymethylcellulose, Celluloseacetatphthalat, oder Polyvinylacetat erhalten werden.

Die Tabletten können auch aus mehreren Schichten bestehen. Entsprechend können Draggées durch Überziehen von analog den Tabletten hergestellten Kernen mit üblicherweise in Dragéeüberzügen verwendeten Mitteln, beispielsweise Kollidon oder Schellack, Gummi arabicum, Talk, Titandioxid oder Zucker, hergestellt werden. Zur Erzielung eines Depotseffekts oder zur Vermeidung von Inkopatibilitäten kann der Kern auch aus mehreren Schichten bestehen. Desgleichen kann auch die Dragéehülle zur Erzielung eines Depotseffekts aus mehreren Schichten bestehen, wobei die oben bei den Tabletten erwähnten Hilfsstoffe verwendeten werden können.

Säfte der Wirkstoffe bzw. Wirkstoffkombinationen können zusätzlich noch ein Süßungsmittel, wie Saccharin, Cyclamat, Gulycerin oder Zucker, sowie ein geschmacksverbesserndes Mittel, z. B. Aromastoffe, wie Vanillin oder Orangenextrakt, enthalten. Sie können außerdem Suspendierhilfsstoffe oder Dickungsmittel, wie Natriumcarboxymethyl-cellulose, Netzmittel, beispielsweise Kondensationsprodukte von Fettalkoholen mit Äthylenoxid, oder Schutzstoffe. wie p-Hydroxybenzoate, enthalten.

Injektionslösungen werden in üblicher Weise, z. B. unter Zusatz von Konservierungsmitteln, wie p-Hydroxybenzoaten, oder Stabilisatoren, wie Komplexonen, hergestellt und in Injektionsflaschen oder Ampullen abgefüllt.

Die Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischer der dafür vorgesehenen Wirkstoffe bzw. Wirkstoffkombinationen mit üblichen Trägermitteln, wie Neutralfetten oder Polyäthylenglykol bzw. dessen Derivaten, herstellen.

Die Verbindungen sind auch für die Kombination mit anderen pharmakodynamisch wirksamen Stoffen wie z. B. Coronardilatatoren, Herzglykosiden oder Tranquilizern geeignet.

Die folgenden Formulierungsbeispiele erläutern die Erfindung, ohne sie zu beschränken:

**Beiepiel 1**: Tabletten

| | |
|---|---|
| Wirkstoff gemäß Erfindung | 50,0 mg |
| Milchzucker | 95,0 mg |
| Maisstärke | 45,0 mg |
| kolloidale Kieselsäure | 2,0 mg |
| lösliche Stärke | 5,0 mg |
| Magnesiumstearat | 3,0 mg |
| | insgesamt 200,0 mg |

**Herstellung**:

Der Wirkstoff wird mit einem Teil der Hilfsstoffe gemischt und mit einer Lösung der löslichen Stärke in Wasser granuliert. Nach dem Trocknen des Granulats wird der Rest der Hilfsstoffe zugemischt und die Mischung zu Tabletten verpreßt.

**Beispiel 2**: Dragées

| | |
|---|---|
| Wirksoff gemäß Erfindung | 20,0 mg |
| Milchzucker | 100,0 mg |
| Maisstärke | 65,0 mg |
| kolloidale Kieselsäure | 2,0 mg |
| lösliche Stärke | 5,0 mg |
| Magnesiumstearat | 3,0 mg |

**Herstellung**:

Der Wirkstoff und die Hilfsstoffe werden, wie in Beispiel 1 beschrieben, zu Tablettenkernen verpreßt, die mit Zucker, Talcum und Gummi arabicum in üblicher Weise dragiert werden.

**Beispiel 3**: Suppositorien

| | |
|---|---|
| Wirkstoff gemäß Erfindung | 50,0 mg |
| Milchzucker | 250,0 mg |
| Suppositorienmasse q. s. ad | 1,7 g |

**Herstellung**:

Der Wirkstoff und der Milchzucker werden miteinander vermischt und die Mischung in der geschmolzenen Suppositorienmasse gleichmäßig suspendiert. Die Suspensionen werden in gekühlte Formen zu Suppositorien von 1,7 g Gewicht ausgegossen.

**Beispiel 4**: Ampullen

| | |
|---|---|
| Wirkstoff gemäß Erfindung | 20,0 mg |
| Natriumchlorid | 5,0 mg |
| Bi-destilliertes Wasser q. s. ad | 2,0 ml |

**Herstellung**:

Der Wirkstoff und das Natriumchlorid werden in bi-destilliertem Wasser gelöst und die Lösung in Ampullen steril abgefüllt.

**Beispiel 5**: Ampullen

| | |
|---|---|
| Wirkstoff gemäß Erfindung | 10,0 mg |
| Natriumchlorid | 7,0 mg |
| Bi-destilliertes Wasser q. s. ad | 1,0 ml |

**Beispiel 6**: Tropfen

| | |
|---|---|
| Wirkstoff gemäß Erfindung | 0,70 g |
| p-Hydroxybenzoesäuremethylester | 0,07 g |
| p-Hydroxybenzoesäurepropylester | 0,03 g |
| Entmineralisiertes Wasser q. s. ad | 100,00 ml |

**Herstellung:**

Der Wirkstoff und die Konservierungsmittel werden in entmineralisiertem Wasser gelöst, die Lösung filtriert und in Flaschen zu je 100 ml abgefült.

## Patentansprüche

Verwendung von 2-[Phenylamino]imidazolinen-(2) der allgemeinen Formel

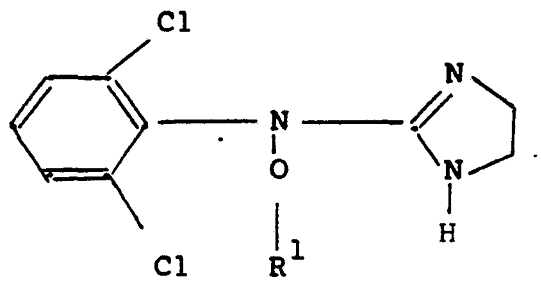

I

in der
$R^1$ eine Methyl, Propyl- oder Isobutylgruppe, eine Hydroxyäthyl-, Hydroxypropyl-, Methoxyäthyl-, Chlorhydroxypropyl-, Benzyl oder Phenäthylgruppe,
bedeutet sowie von deren physiologisch verträglichen Säureadditionssalzen zur Herstellung eines Arzneimittels zur Behandlung von ischämischen Herzerkrankungen und von Sinustachykardien verschiedener Genese.

## Claim

Use of 2-[phenylemino]-2-imidazolines of the general formula

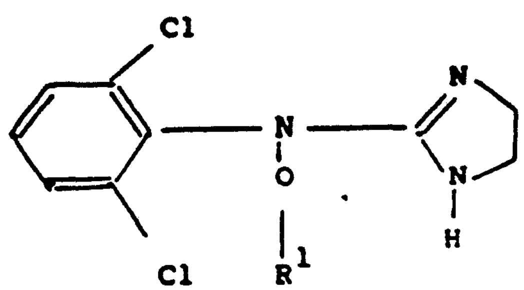

I

in which
$R_1$ denotes a methyl, propyl or isobutyl group, a hydroxyethyl, hydroxypropyl, methoxyethyl, chlorohydroxypropyl, benzyl or phenethyl group,
and of their physiologically tolerated acid addition salts, for the preparation of a medicament for the treetment of ischaemic heart disorders and of sinus tachycardias of various aetiologies.

**Revendication**

Utilisation de 2-[phénylamino]imidazolines-(2) de formule générale

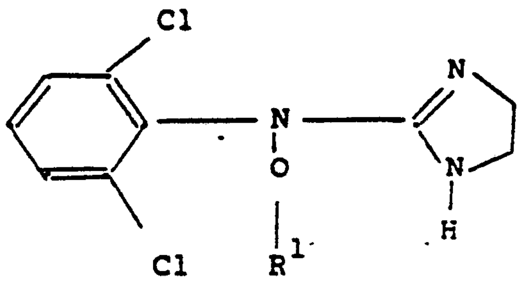

I

dans laquelle

$R^1$ représente un groupe méthyle, propyle ou isobutyle, un groupe hydroxyéthyle, hydroxypropyle, méthoxyéthyle, chlorohydroxypropyle, benzyle ou phénéthyle,

ainsi que de leurs sels d'addition d'acides physiologiquement supportables pour la préparation d'un médicament pour le traitement des maladies cardiaques ischémiques et des tachycardies sinusales de différentes origines.